**Europäisches Patentamt**

**European Patent Office**

**Office européen des brevets**

(19)

(11) Veröffentlichungsnummer : **0 319 555 B1**

(12)

# EUROPÄISCHE PATENTSCHRIFT

(45) Veröffentlichungstag der Patentschrift :
**29.01.92 Patentblatt 92/05**

(51) Int. Cl.$^5$ : **A61L 15/16**

(21) Anmeldenummer : **88904448.3**

(22) Anmeldetag : **27.05.88**

(86) Internationale Anmeldenummer :
**PCT/AT88/00038**

(87) Internationale Veröffentlichungsnummer :
**WO 88/09185 01.12.88 Gazette 88/26**

(54) **TRANSDERMAL THERAPEUTISCH WIRKENDE PHARMAZEUTISCHE ZUBEREITUNG SOWIE VORRICHTUNG ZUM AUFBRINGEN DER ZUBEREITUNG.**

(30) Priorität : **27.05.87 AT 1372/87**

(43) Veröffentlichungstag der Anmeldung :
**14.06.89 Patentblatt 89/24**

(45) Bekanntmachung des Hinweises auf die
Patenterteilung :
**29.01.92 Patentblatt 92/05**

(84) Benannte Vertragsstaaten :
**AT BE CH DE FR GB IT LI LU NL SE**

(56) Entgegenhaltungen :
**EP-A- 54 279**
**DD-A- 217 989**
**DE-A- 2 214 306**
**FR-A- 1 587 321**
**CHEMICAL ABSTRACTS, vol. 102, no. 26, 01**
**July 1985, Columbus, OH (US); p. 347, no.**
**226041a**

(73) Patentinhaber : **Burghart, Kurt, Dr.**
**Sägeberg 8**
**W-2217 Rosdorf (DE)**
Patentinhaber : **Burghart, Walter**
**Salmgasse 4**
**A-1030 Wien (AT)**

(72) Erfinder : **Burghart, Kurt, Dr.**
**Sägeberg 8**
**W-2217 Rosdorf (DE)**
Erfinder : **Burghart, Walter**
**Salmgasse 4**
**A-1030 Wien (AT)**

(74) Vertreter : **Kretschmer, Adolf, Dipl.-Ing. et al**
**Patentanwälte Dipl.Ing. A. Kretschmer Dr.**
**Thomas M. Haffner Schottengasse 3a**
**A-1014 Wien (AT)**

EP 0 319 555 B1

## Beschreibung

Die Erfindung bezieht sich auf eine transdermal therapeutisch wirkende pharmazeutische Zubereitung mit einem den Wirkstoff enthaltenden filmbildenden Polymer, sowie eine Trägermatrix für eine transdermal therapeutische Zubereitung und die Verwendung dieser Trägermatrix.

Derartige Zubereitungen werden häufig auch als transdermal therapeutisches System für Arzneimittelwirkstoffe bezeichnet. Derartige Systeme dienen der kontinuierlichen, dosierten Einbringung von Arzneimittelwirkstoffen, wie z.B. von Nitroglycerin, in den Blutkreislauf. Es sind verschiedene Typen derartiger Systeme bekannt. Sie besitzen im allgemeinen als Kernstück ein den Wirkstoff enthaltendes mikroporöses Kunststoffreservoir. Dieses kann auf der der Haut zugewandten Seite mit einer mikroporösen Membran abgedeckt sein, deren Durchlässigkeit für den Wirkstoff geringer ist als diejenige des Reservoirs und die demgemäß als Steuermembran zur Wirkstoffdosierung dient. Bei einem weiteren bekannten Systemtyp befindet sich der Wirkstoff in porösen Mikrokapseln, die in ein druckempfindliches Klebmittel eingebracht sind und in unmittelbaren Kontakt mit der Haut gebracht werden. Ferner sind Systeme zur transdermalen Applikation von Arzneimittelwirkstoffen bekannt, bei denen der Wirkstoff aus der Kunststoffmatrix durch eine Übertragungsschicht, in der der Wirkstoff eine bestimmte Sättigungslöslichkeit besitzt, auf die Haut aufgebracht wird. In diesem Fall wird die Wirkstoffdosierung durch die Sättigungslöslichkeit des Wirkstoffes in der Übertragungsschicht reguliert.

Es sind weiters bereits versprühbare, filmbildende Polymere als Wund-Spray oder zur Behandlung von örtlichen Krankheiten bekannt. In derartigen filmbildenden Zusammensetzungen können antimikrobielle oder lokal wirkende Wirkstoffe enthalten sein. Diese Systeme werden jedoch nicht genau dosiert auf ein zur Resorption bestimmtes, definiertes Hautareal aufgesprüht und zielen auch nicht auf eine gesteuerte Wirkstofffreigabe aus dem System durch die Haut ab. Der Wirkstoff kann daher systemisch nicht in gesteuerter, über einen längeren Zeitraum einen konstanten Plasmaspiegel liefernder Form zur Wirkung kommen.

Die Erfindung zielt nun darauf ab, eine transdermal therapeutisch wirkende pharmazeutische Zubereitung der eingangs genannten Art zu schaffen, mit welcher die Möglichkeit geschaffen wird einen vorbestimmten Plasmaspiegel für lange Zeiträume, insbesondere über einen Zeitraum von wenigstens 12 Std. zu erzielen und über diesen Zeitraum konstant zu halten. Weiters zielt die Erfindung darauf ab diesen Effekt ohne die Verwendung von üblichen Abdeckungen und anderen aufwendiger zu handhabenden Hilfsmitteln sicherzustellen. Insbesondere soll durch die erfindungsgemäße pharmazeutische Zubereitung die Applikation und damit die korrekte Anwendung erleichtert werden. Zur Lösung dieser Aufgabe besteht die erfindungsgemäße transdermal therapeutisch wirkende pharmazeutische Zubereitung im wesentlichen darin, daß die transdermal therapeutisch wirkende Zubereitung

a) eine polymere flüssige zu einem flexiblen Film aushärtende Matrix, bestehend aus einem Vinylpyrrolidon-Vinylacetat Copolymeren und Polymethacrylsäurebutylester

b) den Wirkstoff

c) ein die Freigabe des Wirkstoffes steuerndes Lösungsmittel, in welchem der Wirkstoff zumindest teilweise oder vollständig löslich ist, gewählt aus der Gruppe Sorbitanmacrogollaurat, Paraffin, mittelkettige Fettsäuredi- oder -triglyceride, Propylencarbonat oder Mischungen davon und

d) ein auf der Haut verdampfendes Lösungsmittel für die Matrix enthält und gemeinsam mit einem Treibmittel in einer Vorrichtung zum dosierten Versprühen auf eine vorgegebene Fläche der Zusammensetzung vorliegt. Dadurch, daß in der Zubereitung ein die Freigabe des Wirkstoffes steuerndes Lösungsmittel, in welchem der Wirkstoff zumindest teilweise löslich ist, in der polymeren flüssigen zu einem flexiblen Film aushärtenden Matrix enthalten ist, kann auf die Verwendung von zusätzlichen Membranen und auf die Verwendung von Abdeckungen verzichtet werden. Eine vorteilhafte polymere flüssige Matrix besteht aus einem Vinylpyrrolidon-Vinylacetat Copolymeren und Polymethacrylsäurebutylester. Eine derartige Matrix zeichnet sich dadurch aus, daß sie mit Wasser abwaschbar ist und nach dem Aushärten eine hinreichende Abriebfestigkeit und mechanische Stabilität des flexiblen Filmes gewährleistet. Als die Freigabe des Wirkstoffes steuerndes Lösungsmittel bzw. Lösungsmittelgemisch, in welchem der Wirkstoff teilweise löslich ist, kommen in erster Linie Sorbitanmacrogollaurat, Paraffin, mittelkettige Fettsäuredi- und -triglyceride, Propylencarbonat oder Mischungen davon in Betracht. Durch das auf der Haut verdampfende Lösungsmittel für die Matrix wird die Aushärtung zu einem flexiblen Film beschleunigt. Dadurch, daß die Zubereitung so formuliert ist, daß sie gemeinsam mit einem Treibmittel in einer Vorrichtung zum dosierten Versprühen vorliegen kann, wird die Handhabung und Applikation wesentlich vereinfacht und es wird mit der Vorrichtung zum dosierten Versprühen auch eine vorgegebene Fläche sicher eingehalten. Es wird gleichzeitig sichergestellt, daß durch die transdermale Steuerung im System überhöhte Wirkstoffkonzentrationen vermieden werden, da je nach vorgewählter Fläche und Dosierung die jeweilig maximal zulässigen Konzentrationen des Wirkstoffes im Blut sicher beherrscht werden und über einen langen Zeitraum konstant gehalten werden kann.

2

Mit Vorteil liegen hierbei das Vinylpyrrolidon-Vinylacetat Copolymer und Polymethacrylsäurebutylester in einem Mischungsverhältnis von 3:1 bis 1:3 vor. In besonders bevorzugter Weise liegen das Vinylpyrrolidon-Vinylacetat Copolymer und Polymethacrylsäurebutylester in einem Mischungsverhältnis von 1,3:1 bis 1:1,3 vor, wobei bei steigenden Polymethacrylsäurebutylesteranteilen die auf der Haut gebildeten Filme durch Abziehen leicht entfernt werden können.

Der Zusatz von Polymethacrylsäurebutylester zu Vinylpyrrolidon-Vinylacetat Copolymer in den oben angegebenen Mengenverhältnissen dient hierbei in erster Linie der Erzielung einer hinreichenden Abriebfestigkeit. Es sind prinzipiell auch andere Polymere, wie beispielsweise Schellack, Eudragite oder HPMCP geeignet, jedoch ist mit Rücksicht auf den Treibgaszusatz darauf zu achten, daß die Polymerzusätze nicht aus der Lösung ausfallen sollen. Eine weitere Beschränkung für den Einsatz derartiger polymerer Zusätze ist dadurch gegeben, daß die Düse des Sprühkopfes nicht verkleben soll. Insbesondere der Einsatz von Ethylzellulose hat sich aus diesem Grunde nicht als Vorteilhaft erwiesen, da Ethylzellulose dazu neigt, die Düse des Sprühkopfes zu verkleben.

Als Wirkstoffe kommen eine Reihe von Wirkstoffen in Betracht, für welche eine kontinuierliche Abgabe wünschenswert erscheint. Im besonderen sind Wirkstoffe, die durch die Haut permeieren, bei niedrigen Plasmaspiegeln starke physiologische Wirkung zeigen und niedrige biologische Halbwertszeiten besitzen wie insbesondere Nitroglycerin, besonders vorteilhaft im Rahmen der erfindungsgemäßen pharmazeutischen Zubereitung einsetzbar. Darüberhinaus können jedoch Wirkstoffe gegen Reisekrankheit, wie Tropasäureester bzw. Scopolamin, Betablocker wie Propanolol, Migränemittel wie Methysergid, Antihypertonika, wie Clonidin und Reserpin, Hormone, wie Östradiol, Analgetika, wie Fentanyl, Aspirin, Ibuprofen, Piroxicam und Phenylbutazon, Lokalanästhetika, wie Procain und Lidocain, Calciumantagonisten und Herzkreislaufmittel, wie Nifedipin, Nicardipin und Molsidomin, Sedativa, wie Phenobarbital und Cyclobarbital oder aber auch Nicotin, Antitumoragentien, wie Krestin und Ancitabin, Enzyme, wie Lysozym als Wirkstoffe eingesetzt werden. Im Falle der Verwendung von Nitroglycerin als Wirkstoff wird mit Vorteil so vorgegangen, daß Nitroglycerin als 10%ige, alkoholische Lösung oder in Fettsäuredi- und -triglyceriden oder Mischungen davon gelöst in der Zusammensetzung vorliegt.

Besonders günstige Werte für die gewünschten Plasmaspiegel können insbesondere dadurch erreicht werden, daß der Wirkstoff und das die Freigabe des Wirkstoffes steuernde Lösungsmittel bzw. Lösungsmittelgemisch im Verhältnis von 2:1 bis 1:2 vorliegen. In besonders vorteilhafter Weise wird das Verhältnis von Wirkstoff zu dem die Freigabe des Wirkstoffes steuernden Lösungsmittel von 1:1 bis 1:1,5 gewählt.

Um sicherzustellen, daß der Film nach dem Aufsprühen auf der Haut rasch trocknet, werden Lösungsmittel mit niedrigem Siedepunkt bevorzugt. Derartige Lösungsmittel können aus der Gruppe Dichlormethan, Ethanol, Ethylacetat, Isopropanol oder Mischungen davon ausgewählt sein, wobei insbesondere die Verwendung von Ethylacetat gleichzeitig das Sprühverhalten verbessert, da ein Spritzen vermieden wird. Mit Ethylacetat lassen sich besonders homogene Filme auf der Haut ausbilden.

Als Treibmittel kann in den erfindungsgemäßen Präparaten jede flüchtige organische Verbindung mit einer Siedetemperatur bei atmosphärischem Druck unterhalb 7°C, die gegenüber den möglicherweise im Präparat anwesenden Zusätzen inert ist, verwendet werden. Beispielsweise können Kohlenwasserstoffe, Propan, Butan, Isobutan, halogenierte Kohlenwasserstoffe, Fluorchlorkohlenwasserstoffe, wie Dichlor-difluormethan, sowie Dimethylether, sowie deren Gemische verwendet werden.

Mit Rücksicht auf die oben angeführten Beschränkungen im Einsatz der Bestandteile der polymeren Matrix bei gleichzeitigem Vorliegen eines Treibmittels wird mit Vorteil das Verhältnis von polymerer Matrix zu Lösungs- und Treibmittel im Bereich von 1:10 bis 1:15 eingestellt. In besonders bevorzugter Weise wird das Verhältnis von polymerer Matrix zu Lösungs- und Treibmittel im Bereich von 1:11 bis 1:13 eingestellt. Durch Einhalten eines derartigen Mengenverhältnisses wird sichergestellt, daß die Sprühdose nicht durch rasch aushärtende Polymerpartikel verstopft bzw. teilweise verlegt wird. Dies ist vor allem deshalb wichtig, da bereits mit einer nur zu einem Teil verlegten Sprühdose kein homogener, die gesamte zu besprühende Fläche bedeckender Film ausgebildet werden kann.

Insbesondere die Verwendung von Sorbitanmacrogollaurat als die Freigabe des Wirkstoffes steuerndes Lösungsmittel hat hiebei die vorteilhafte Nebenwirkung, daß durch Wahl der entsprechenden Menge dieses Lösungsmittels der Fluß in weiten Grenzen einstellbar ist.

Um ein gutes Aushärten der Zubereitung sicherzustellen, sind mit Vorteil in der pharmazeutischen Zubereitung maximal 35% des die Freigabe des Wirkstoffes steuernden Lösungsmittels bzw. Lösungsmittelgemisches in bezug auf die polymere flüssige zu einem flexiblen Film aushärtende Matrix enthalten. Durch Einhalten derartiger Mengen an dem die Freigabe des Wirkstoffes steuernden Lösungsmittels bzw. Lösungsmittelgemisches in bezug auf die polymeren Bestandteile wird sichergestellt, daß die pharmazeutische Zubereitung zu einem nicht klebrigen, abriebfesten Film aushärtet.

Die erfindungsgemäße transdermal therapeutische Zubereitung ist vorzugsweise dadurch gekennzeichnet, daß in der Zubereitung 2 bis 10% der zu einem flexiblen Film aushärtenden polymeren Bestandteile, 15 bis 50% der Lösungsmittel mit niedrigem Siedepunkt, 0,5 bis 5% des die Freigabe des Wirkstoffes steuernden Lösungsmittels bzw. Lösungsmittelgemisches und 50 bis 80% des Treibmittels sowie der Wirkstoff enthalten sind.

Die erfindungsgemäße pharmazeutische Zubereitung kann in besonders einfacher Weise hergestellt werden, da hiefür nur die Bestandteile in den entsprechenden Mengenverhältnissen vermischt werden müssen und das transdermal therapeutische System erst nach der gezielten Applikation auf der Haut, durch Verdampfen der leicht flüchtigen Lösungsmittel und Ausbildung des Filmes entsteht. Dieses Herstellungsverfahren zeichnet sich gegenüber bekannten Herstellungsverfahren für transdermal therapeutische Systeme dadurch aus, daß keine aufwendige technische Vorfertigung des Systems erforderlich ist.

Insgesamt ergibt sich durch die Verwendung der erfindungsgemäßen Zubereitung in Form eines Sprays bzw. Aerosols eine besonders einfache Herstellung und eine gegenüber bekannten transdermal therapeutischen Systemen bessere Verträglichkeit, da der Film, welcher auf der Haut verbleibt und keine besondere Abdeckung benötigt, extrem dünn gehalten werden kann und dadurch die Atmung der Haut und insbesondere den Gas- und Wasseraustausch mit der Umgebung kaum beeinträchtigt. Aufgrund des dünnen Filmes ist auch ein geringerer Wärmestau gegenüber bekannten Systemem vorhanden, und auch der subjektive Komfort im Vergleich zu dicken und schweren Pflastern wesentlich größer. Der polymere flexible Film kann darüberhinaus durchsichtig ausgebildet sein, so daß er auch nicht ohne weiteres zu erkennen ist. Die rasch abdampfenden Lösungsmittel für den polymeren Film wirken vor ihrem Verdampfen als Penetrationsverstärker, so daß eine schnellere Wirkstoffanflutung nach der Applikation beobachtet werden kann.

Mit Rücksicht auf die einfache Applikation ist auch eine individuellere Dosierung ohne weiteres möglich, da zur linderung der Dosierung lediglich die Anzahl der Sprühstöße vor dem jeweilig gewünschten Zeiträumen geändert werden müßten. Die Verwendung der erfindungsgemäßen polymeren Matrix erlaubt auch die leichte rückstandlose Entfernung des Filmes, beispielsweise unter Anwendung von lauwarmen Wasser oder durch Abziehen des Films.

Die Trägermatrix für eine transdermal therapeutische Zubereitung ist im wesentlichen dadurch gekennzeichnet, daß sie polymere, zu einem flexiblen Film aushärtende Bestandteile wie Vinylpyrrolidon/Vinylacetat Compolymere und Polymethacrylsäurebutylester, ein die Freigabe des in der Zubereitung enthaltenen Wirkstoffes steuerndes Lösungsmittel bzw. Lösungsmittelgemisch, ausgewählt aus der Gruppe Sorbitanmacrogollaurat, Paraffin, mittelkettige Fettsäuredi- oder -triglyceride, Propylencarbonat oder Mischungen davon, und auf der Haut schnell verdampfende die Filmbildung fördernde Lösungsmittel, ausgewählt aus der Gruppe Ethanol, Dichlormethan, Ethylacetat, Isopropanol oder Mischungen davon, enthält und daß darin 2 bis 10% der zu einem flexiblen Film aushärtenden polymeren Bestandteile, 15 bis 50% der Lösungsmittel mit niedrigem Siedepunkt, 0,5 bis 5% des die Freigabe des Wirkstoffes steuernden Lösungsmittels bzw. Lösungsmittelgemisches und 50 bis 80% des Treibmittels enthalten sind. Eine derartige Trägermatrix ist in besonders bevorzugter Weise zur Verwendung für die Herstellung eines transdermal therapeutischen Pharmazeutikums geeignet. Die erfindungsgemäße Trägermatrix ergibt nach der Applikation auf die Haut einen besonders rasch aushärtenden homogenen Film, der durch Reiben nicht von der Haut entfernt werden kann. Der aus der Trägermatrix gebildete flexible Film läßt sich jedoch unter Anwendung von lauwarmen Wasser leicht abwaschen oder direkt abziehen.

Die Erfindung wird nachfolgend an Hand von Ausführungsbeispielen näher erläutert.

Beispiel 1:

In einer Braunglasflasche, die mit einem 300 mg Dosierventil versehen war, wurde ein Aerosol folgender Zusammensetzung hergestellt:

```
10%            ethanolische          Nitroglycerinlösung
40,0 mg
Polymethacrylsäurebutylester                (MG:100 000)
11,0 mg
Vinylpyrrolidon/Vinylacetat      Copolymer      (MG:50 000)
10,0 mg
Ethanol
5,0 mg
Methylenchlorid
38,0 mg
Sorbitanmacrogollaurat
5,0 mg
Treibgas                                         191,0 mg
                                                 300,0 mg
```

Ein Aerosol dieser Zusammensetzung ließ sich gut versprühen und bildete auf der Haut einen gleichmäßigen Film. Ein derartiger Film konnte durch Reiben nicht entfernt werden, ließ sich aber mit warmen Wasser (ca. 35°C) leicht entfernen.

Beispiel 2:

In einer Braunglasflasche, die mit einem 300 mg Dosierventil versehen war, wurde ein Aerosol folgender Zusammensetzung hergestellt:

```
10% ethanolische Nitroglycerinlösung               40,0 mg
Polymethacrylsäurebutylester (MG:100 000)          11,0 mg
Vinylpyrrolidon/Vinylacetat Copolymer (MG:50 000)  10,0 mg
Ethanol                                             5,0 mg
Methylenchlorid                                     38,0 mg
mittelkettige Fettsäuredi- und/oder
-triglyceride                                        5,0 mg
Treibgas                                           191,0 mg
                                                   295,0 mg
```

Ein Aerosol dieser Zusammensetzung ließ sich gut versprühen und bildete auf der Haut einen gleichmäßigen Film. Ein derartiger Film konnte durch Reiben nicht entfernt werden, ließ sich aber mit warmen Wasser (ca. 35°C) leicht entfernen.

Beispiel 3:

In einer Braunglasflasche, die mit einem 300 mg Dosierventil versehen war, wurde ein Aerosol folgender Zusammensetzung hergestellt:

5

| | |
|---|---:|
| 10% ethanolische Nitroglycerin | 40,0 mg |
| Polymethacrylsäurebutylester (MG:100 000) | 11,0 mg |
| Vinylpyrrolidon/Vinylacetat Copolymer (MG:50 000) | 10,0 mg |
| Ethanol | 5,0 mg |
| Methylenchlorid | 38,0 mg |
| Treibgas | 191,0 mg |
| | 300,0 mg |

Ein Aerosol dieser Zusammensetzung ließ sich gut versprühen und bildete auf der Haut einen gleichmäßigen Film. Ein derartiger Film konnte durch Reiben nicht entfernt werden, ließ sich aber mit warmen Wasser (ca. 35°C) leicht entfernen.

Beispiel 4:

In einer Braunglasflasche, die mit einem 300 mg Dosierventil versehen war, wurde ein Aerosol folgender Zusammensetzung hergestellt:

| | |
|---|---:|
| 10% ethanolische Nitroglycerinlösung | 40,0 mg |
| Polymethacrylsäurebutylester (MG:100 000) | 11,0 mg |
| Vinylpyrrolidon/Vinylacetat Copolymer (MG.50 000) | 10,0 mg |
| Ethanol | 5,0 mg |
| Methylenchlorid | 38,0 mg |
| Paraffin | 5,0 mg |
| Treibgas | 191,0 mg |
| | 300,0 mg |

Ein Aerosol dieser Zusammensetzung ließ sich gut versprühen und bildete auf der Haut einen gleichmäßigen Film. Ein derartiger Film konnte durch Reiben nicht entfernt werden, ließ sich aber mit warmen Wasser (ca. 35°C) leicht entfernen.

Beispiel 5:

In einer Braunglasflasche, die mit einem 300 mg Dosierventil versehen war, wurde ein Aerosol folgender Zusammensetzung hergestellt:

| | |
|---|---:|
| 10% ethanolische Nitroglycerinlösung | 40,0 mg |
| Polymethacrylsäurebutylester (MG:100 000) | 11,0 mg |
| Vinylpyrrolidon/Vinylacetat Copolymer (MG:50 000) | 10,0 mg |
| Ethanol | 5,0 mg |
| Methylenchlorid | 38,0 mg |
| Ethylacetat | 30,0 mg |
| Sorbitanmacrogollaurat | 5,0 mg |
| Treibgas | 161,0 mg |
| | 300,0 mg |

EP 0 319 555 B1

Ein Aerosol dieser Zusammensetzung ließ sich gut versprühen und bildete auf der Haut einen gleichmäßigen Film. Ein derartiger Film konnte durch Reiben nicht entfernt werden, ließ sich aber mit warmen Wasser (ca. 35°C) leicht entfernen.

Beispiel 6:

In einer Braunglasflasche, die mit einem 300 mg Dosierventil versehen war, wurde ein Aerosol folgender Zusammensetzung hergestellt:

```
10% ethanolische Nitroglycerinlösung                      80,0 mg
Polymethacrylsäurebytylester (MG:100 000)                 11,0 mg
Vinylpyrrolidon/Vinylacetat Copolymer (MG:50 000)         10,0 mg
Ethanol                                                    5,0 mg
Methylenchlorid                                           38,0 mg
Ethylacetat                                               30,0 mg
Sorbitanmacrogollaurat                                     5,0 mg
Treibgas                                                  121,0 mg
                                                         300,0 mg
```

Ein Aerosol dieser Zusammensetzung ließ sich gut versprühen und bildete auf der Haut einen gleichmäßigen Film. Ein derartiger Film konnte durch Reiben nicht entfernt werden, ließ sich aber mit warmen Wasser (ca. 35°C) leicht entfernen.

Beispiel 7:

In einer Braunglasflasche, die mit einem 300 mg Dosierventil versehen war, wurde ein Aerosol folgender Zusammensetzung hergestellt:

```
10% ethanolische Nitroglycerinlösung                      40,0 mg
Polymethacrylsäurebutylester (MG:100 000)                 16,0 mg
Vinylpyrrolidon/Vinylacetat Copolymer (MG:50 000)          5,0 mg
Ethanol                                                    5,0 mg
Methylenchlorid                                           38,0 mg
Ethylacetat                                               30,0 mg
Sorbitanmacrogollaurat                                     5,0 mg
Treibgas                                                  161,0 mg
                                                         300,0 mg
```

Ein Aerosol dieser Zusammensetzung ließ sich gut versprühen und bildete auf der Haut einen gleichmäßigen Film. Ein derartiger Film konnte durch Reiben nicht entfernt werden, ließ sich aber mit warmen Wasser (ca. 35°C) leicht entfernen, oder direkt abziehen.

Mit der Rezeptur nach Beispiel 1 wurde ein Resorptionsversuch an Probanden durchgeführt und der Wirkstofffluß nach 3, 6, 9 und 12 Stunden bestimmt. Dafür wurde ein Sprühstoß von 150 mg, der 2 mg Wirkstoff enthielt, auf eine Aluminiumfolie gesprüht. Die besprühte Fläche betrug genau 5 cm². Nach dem Trocknen des Filmes wurde die Folie mit dem Film gewogen und anschließend die Hälfte der Folie auf die Haut gebracht. Aus der zweiten Folienhälfte wurde der Wirkstoffgehalt bestimmt. Nach 3 Stunden wurde die erste Folienhälfte von der Haut abgelöst und die resorbierte Wirkstoffmenge bestimmt. Danach wurden analoge Versuche für die Zeiträume von 6, 9 und 12 Stunden durchgeführt. Aus der jeweiligen resorbierten Wirkstoffmenge läßt sich der Fluß an Nitroglycerin durch die Haut bestimmen.

7

Der Resorptionsversuch zeigte folgendes Ergebnis:

| Zeit | Wirkstofffluß |
|------|---------------|
| 3 Stunden | 11,9 $\mu g/cm^2/Std.$ |
| 6 Stunden | 11,4 $\mu g/cm^2/Std.$ |
| 9 Stunden | 12,6 $\mu g/cm^2/Std.$ |
| 12 Stunden | 7,5 $\mu g/cm^2/Std.$ |

Ein therapeutisch wirkender Wirkstofffluß sollte in der Größenordnung von 15 bis 25 $\mu g/cm^2/Std.$ liegen. Ein Wirkstofffluß in dieser Größenordnung stellt bei einer bedeckten Fläche von 10 $cm^2$, eine ausreichende Plasmakonzentration des Wirkstoffes sicher. Die in dem angeführten Resorptionsversuch gefundenen Werte lagen durchwegs unter den gewünschten Werten, zeichneten sich aber über einen Zeitraum von mindestens 9 Stunden durch große Konstanz aus. Da aber die Wirkstoffzusammensetzung nur auf eine Fläche von 5 $cm^2$ aufgebracht wurde, läßt sich unschwer erkennen, daß bei Aufbringen von vier Sprühstößen à 5 $cm^2$ ein im therapeutisch wirksamen Bereich liegender Plasmaspiegel erzielt werden kann. Auch der deutlich niedrigere Wert für den Wirkstofffluß nach 12 Stunden läßt sich mit der geringen bei den Probandenversuchen auf die Haut aufgebrachten Wirkstoffmenge begründen, da das Wirkstoffreservoir zu diesem Zeitpunkt bereits zu etwa 60% verbraucht war. Eine Konstanz des Wirkstoffflusses bis zu einem Zeitraum von 24 Stunden wurde bei diesem Versuch nicht angestrebt, da Nitroglycerin als Wirkstoff der Toleranzentwicklung unterliegt und dadurch die Zubereitung im Laufe der Zeit an therapeutischer Wirkung verliert, so daß eine Dosisreduktion, beispielsweise in der Nacht, wünschenswert erscheint.

Eine weitere Steigerung des Wirkstoffflusses in der Anflutungsphase kann mit Sicherheit dadurch erreicht werden, daß die pharmazeutische Zubereitung direkt auf die Haut versprüht wird.

In einem zweiten Resorptionsversuch an Probanden, wurden die pharmazeutischen Zubereitungen nach den Beispielen 1 bis 4 in oben beschriebener Weise versprüht und appliziert. Nach 6 Stunden wurde die erste Hälfte wiederum abgelöst und die resorbierte Wirkstoffmenge bestimmt. Aus dieser resorbierten Wirkstoffmenge läßt sich wiederum der Wirkstofffluß bestimmen. Hiebei wurde mit der Rezeptur nach Beispiel 1 ein Fluß von 11,83 $\mu g/cm^2/Std.$ erreicht, mit der Rezeptur nach Beispiel 2 ein Fluß von 15,07 $\mu g/cm^2/Std.$, mit der Rezeptur nach Beispiel 3 ein Fluß von 3,52 $\mu g/cm^2/Std.$ und mit der Rezeptur nach Beispiel 4 ein Fluß von 7,1 $\mu g/cm^2/Std.$ Da auch bei diesen Versuchen nur ein Sprühstoß auf 5 $cm^2$ Fläche abgegeben wurde und in diesem Sprühstoß jeweils nur etwa 2 mg der Substanz enthalten waren, läßt sich wiederum durch Verdoppelung bis Vervierfachung der mit der Zubereitung besprühten Fläche eine Plasmakonzentration erreichen, die im therapeutisch wirksamen Bereich liegt. Dies gilt insbesondere für die Rezepturen nach Beispiel 1 und 2, wohingegen die Rezepturen nach Beispiel 3 und 4 auch bei Vervierfachung der mit der Zubereitung besprühten Fläche und damit durch Vervierfachung der Wirkstoffmenge kein im therapeutisch wirksamen Bereich liegender Plasmaspiegel erreicht werden kann.

Aus diesen Ergebnissen läßt sich ersehen, daß mit den unterschiedlichen Lösungsmitteln eine gezielte Steuerung des Wirkstoffflusses erreicht werden kann. Als besonders geeignete, die Freigabe des Wirkstoffes steuernde Lösungsmittel haben sich Sorbitanmacrogollaurat und die mittelkettigen Fettsäuredi- und/oder -triglyceride, die in den Rezepturen nach Beispiel 1 und 2, enthalten sind, erwiesen. Mit dem in Beispiel 4 angegebenen Paraffin kann nur ein etwa halb so großer Wirkstofffluß erreicht werden, und die Werte, die in Abwesenheit von derartigen, die Freigabe des Wirkstoffes steuernden Lösungsmitteln erreicht werden können, liegen nur im Bereich von 3,5 $\mu g/cm^2/Std.$ und damit weit unter einem therapeutisch anwendbaren Fluß.

**Patentansprüche**

1. Transdermal therapeutisch wirkende pharmazeutische Zubereitung mit einem den Wirkstoff enthaltenden filmbildenden Polymer, dadurch gekennzeichnet, daß die transdermal therapeutisch wirkende Zubereitung
a) eine polymere flüssige zu einem flexiblen film aushärtende Matrix, bestehend aus einem Vinylpyrrolidon-Vinylacetat Copolymeren und Polymethacrylsäurebutylester
b) den Wirkstoff
c) ein die Freigabe des Wirkstoffes steuerndes Lösungsmittel, in welchem der Wirkstoff teilweise oder vollständig löslich ist, gewählt aus der Gruppe Sorbitanmacrogollaurat, Paraffin, mittelkettige Fettsäuredi- oder

EP 0 319 555 B1

-triglyceride, Propylencarbonat oder Mischungen davon und

d) ein auf der Haut verdampfendes Lösungsmittel für die Matrix

enthält und gemeinsam mit einem Treibmittel in einer Vorrichtung zum dosierten Versprühen auf eine vorgegebene fläche der Zusammensetzung vorliegt.

2. Pharmazeutische Zubereitung nach Anspruch 1 oder 2, dadurch gekennzeichnet, daß das Vinylpyrrolidon-Vinylacetat Copolymer und Polymethacrylsäurebutylester in einem Mischungsverhältnis von 3:1 bis 1:3 vorliegen.

3. Pharmazeutische Zubereitung nach Anspruch 2, dadurch gekennzeichnet, daß das Vinylpyrrolidon-Vinylacetat Copolymer und Polymethacrylsäurebutylester in einem Mischungsverhältnis von 1,3:1 bis 1:1,3 vorliegen.

4. Pharmazeutische Zubereitung nach Anspruch 1, 2 oder 3, dadurch gekennzeichnet, daß der Wirkstoff ein gefäßerweiterndes Mittel und insbesondere Nitroglycerin ist.

5. Pharmazeutische Zubereitung nach Anspruch 1 bis 4, dadurch gekennzeichnet, daß Nitroglycerin als 10%ige, alkoholische Lösung oder in Fettsäuredi- oder -triglyceriden oder Mischungen davon gelöst in der Zusammensetzung vorliegt.

6. Pharmazeutische Zubereitung nach Anspruch 1 bis 6, dadurch gekennzeichnet, daß der Wirkstoff und das die freigabe des Wirkstoffes steuerndes Lösungsmittel im Verhältnis von 2:1 bis 1:2 vorliegen.

7. Pharmazeutische Zubereitung nach Anspruch 6, dadurch gekennzeichnet, daß der Wirkstoff und das die freigabe des Wirkstoffes steuernde Lösungsmittel im Verhältnis von 1:1 bis 1:1,5 vorliegen.

8. Pharmazeutische Zubereitung nach Anspruch 1 bis 7, dadurch gekennzeichnet, daß das (die) auf der Haut verdampfende(n) Lösungsmittel für die Matrix aus der Gruppe Dichlormethan, Ethanol, Ethylacetat, Isopropanol oder Mischungen davon ausgewählt ist (sind).

9. Pharmazeutische Zubereitung nach Anspruch 1 bis 8, dadurch gekennzeichnet, daß das Verhältnis von polymerer Matrix zu Lösungs- und Treibmittel im Bereich von 1:10 bis 1:15 liegt.

10. Pharmazeutische Zubereitung nach Anspruch 9, dadurch gekennzeichnet, daß das Verhältnis von polymerer Matrix zu Lösungs- und Treibmittel im Bereich von 1:11 bis 1:13 liegt.

11. Pharmazeutische Zubereitung nach Anspruch 1 bis 10, dadurch gekennzeichnet, daß in der pharmazeutischen Zubereitung maximal 35% des die freigabe des Wirkstoffes steuernden Lösungsmittels bzw. Lösungsmittelgemisches in bezug auf die polymere flüssige zu einem flexiblen Film aushärtende Matrix enthält.

12. Pharmazeutische Zubereitung nach einem der Ansprüche 1 bis 11, dadurch gekennzeichnet, daß in der Zubereitung 2 bis 10% der zu einem flexiblen film aushärtenden polymeren Bestandteile, 15 bis 50% der Lösungsmittel mit niedrigem Siedepunkt, 0,5 bis 5% des die Freigabe des Wirkstoffes steuernden Lösungsmittels bzw. Lösungsmittelgemisches und 50 bis 80% des Treibmittels sowie der Wirkstoff enthalten sind.

13. Trägermatrix für eine transdermal therapeutische Zubereitung für die Applikation als Spray, dadurch gekennzeichnet, daß sie polymere, zu einem flexiblen Film aushärtende Bestandteile wie Vinylpyrrolidon/Vinylacetat Copolymere und Polymethacrylsäurebutylester, ein die Freigabe des in der Zubereitung enthaltenen Wirkstoffes steuerndes Lösungsmittel bzw. Lösungsmittelgemisch, ausgewählt aus der Gruppe Sorbitanmacrogollaurat, Paraffin, mittelkettige Fettsäuredioder -triglyceride, Propylencarbonat oder Mischungen davon, und auf der Haut schnell verdampfende die filmbildung fördernde Lösungsmittel, ausgewählt aus der Gruppe Ethanol, Dichlormethan, Ethylacetat, Isopropanol oder Mischungen davon, enthält.

14. Trägermatrix für eine transdermal therapeutische Zubereitung für die Applikation als Spray nach Anspruch 13, dadurch gekennzeichnet, daß in der Trägermatrix 2 bis 10% der zu einem flexiblen film aushärtenden polymeren Bestandteile, 15 bis 50% der Lösungsmittel mit niedrigem Siedepunkt, 0,5 bis 5% des die freigabe des Wirkstoffes steuernden Lösungsmittels bzw. Lösungsmittelgemisches und 50 bis 80% des Treibmittels enthalten sind.

15. Verwendung einer Trägermatrix nach Anspruch 13 oder 14, für die Herstellung eines transdermal therapeutischen Pharmazeutikums.


**Claims**

1. Pharmaceutical composition having transdermal therapeutic activity and comprising a film-forming polymer containing the active substance, characterized in that the transdermal therapeutically active composition contains

a) a polymeric liquid matrix curing to form a flexible film, consisting of a vinylpyrrolidone-vinylacetate-copolymer and a polymethacrylic acid-butylester,

b) the active substance

c) a solvent controlling the release of the active substance, in which solvent the active substance is partially

9

or completely soluble, which solvent is selected from the group consisting of sorbitan macrogollaurate, paraffin, diglycerides or triglycerides of fatty acids having medium chain length, propylene carbonate or mixtures thereof, and

d) a solvent for the matrix evaporating on the skin

and is present, together with a propelling agent, within a device for dosed spraying the composition onto a preselected area.

2. Pharmaceutical composition according to claim 1, characterized in that the vinylpyrrolidone-vinylacetate-copolymer and the polymethacrylic acid-butylester are present in a mixing ratio between 3:1 and 1:3.

3. Pharmaceutical composition according to claim 2, characterized in that the vinylpyrrolidone-vinylacetate-copolymer and the polymethacrylic acid-butylester are present in a mixing ratio between 1,3:1 and 1:1,3.

4. Pharmaceutical composition according to any of the claims 1, 2, or 3, characterized in that the active substance is a vasodilatory agent and is in particular nitroglycerol.

5. Pharmaceutical composition according to any of the claims 1 to 4, characterized in that the nitroglycerol is present in the solution as 10% alcoholic solution or as a solution of diglycerides- or -triglycerides of fatty acids or as mixtures thereof.

6. Pharmaceutical composition according to any of the claims 1 to 5, characterized in that the active substance and the solvent controlling the release of the active substance are present in a ratio of 2:1 to 1:2.

7. Pharmaceutical composition according to claim 6, charcterized in that the active substance and the solvent controlling the release of the active substance are present in a ratio of 1:1 to 1:1,5.

8. Pharmaceutical composition according to one of the claims 1 to 7, characterized in that the solvent(s) for the matrix evaporating on the skin is (are) selected from the group consisting of dichloromethane, ethanol, ethylacetate, isopropanol or mixtures therof.

9. Pharmaceutical composition according to any of the claims 1 to 8, characterized in that the ratio of polymeric matrix to solvents and propelling agent is within the range of 1:10 to 1:15.

10. Pharmaceutical composition according to claim 9, characterized in that the ratio of polymeric matrix to solvents and propelling agent is within the range of 1:11 to 1:13.

11. Pharmaceutical composition according to any of the claims 1 to 10, characterized in that in the pharmaceutical composition there is contained the solvent or, respectively, solvent mixture controlling the release of the active substance in a maximum amount of 35% as based on the liquid matrix curing to form a flexible film.

12. Pharmaceutical composition according to any of the claims 1 to 11, charcterized in that in the composition there are contained 2 to 10% of the polymeric constituents curing to form a flexible film, 15 to 50% of the solvents having a low boiling point, 0,5 to 5% of the solvent or, respectively, solvent mixture controlling the release of the active substance and 50 to 80% of the propelling agent as well as the active substance.

13. Carrier matrix for a transdermal therapeutic composition for the application as a spray, characterized in that it contains polymeric constituents curing to form a flexible film, such as vinylpyrrolidone-vinylacetate-copolymers and polymethacrylic acid-butylester, a solvent or, respectively, a solvent mixture controlling the release of the active substance, selected from the group consisting of sorbitan macrogollaurate, paraffin, diglycerides or triglycerides of fatty acids having medium chain length, propylene carbonate or mixtures thereof, and solvents promoting the film formation and rapidly evaporating on the skin, selected from the group consisting of ethanol, dichloromethane, ethylacetate, isopropanol or mixtures thereof.

14. Carrier matrix for a transdermal therapeutic composition for the application as a spray, according to claim 13, characterized in that there are contained in the carrier matrix 2 to 10% of polymeric constituents curing to form a flexible film, 15 to 50% of the solvents having a low boiling point, 0,5 to 5% of the solvent or, respectively, solvent mixtures controlling the release of the active substance and 50 to 80% of the propelling agent.

15. Use of a carrier matrix according to claim 13 or 14 for the production of a transdermal therapeutic pharmaceuticum.

## Revendications

1. Composition pharmaceutique qui a une activité transdermal thérapeutique avec un polymère qui peut former un film et qui contiens la matière active, characterisée en ce que la composition pharmaceutique qui a une activité transdermal thérapeutique contiens

a) une matrice polymère liquide, qui durcisse à un film flexible, consistant d'un copolymère de vinylpyrrolidone et de vinylacetate et de l'ester butylique de l'acide polyméthacrylique

b) la matière active

c) un solvent qui commande la libération de la matière active, où la matière active est soluble partiellement ou complètement, qui est selectionné de la groupe consistant du macrogollaurate de sorbitane, paraffine, diglycérides ou triglycérides de l'acide gras à medium chaine, propylene carbonate ou mixtures de ces solvents et

d) un solvent pour la matrice qui s'évapore sur la peau

et qui est contenu avec un propellant dans un récipient pour l'atomisation défini de la composition sur une surface prédéfini.

2. Composition pharmaceutique selon la revendication 1, characterisée en ce que le rapport des agrégats du copolymère de vinylpyrrolidone et de vinylacetate et de l'ester butylique de l'acide polyméthacrylique est entre 3:1 et 1:3.

3. Composition pharmaceutique selon la revendication 2, characterisée en ce que le rapport des agrégats du copolymère de vinylpyrrolidone et de vinylacetate et de l'ester butylique de l'acide polyméthacrylique est entre 1,3:1 et 1:1,3.

4. Composition pharmaceutique selon l'une des revendications 1, 2 ou 3, characterisée en ce que la matière active est une matière vasodilatorique et est en particulière le nitroglycérine.

5. Composition pharmaceutique selon l'une des revendications 1, à 4, characterisée en ce que le nitroglycérine est contenu dans la solution comme solution alcoolique à 10% ou comme solution de diglycérides ou triglycérides de l'acide gras à medium chaine ou mixtures de ces substances.

6. Composition pharmaceutique selon l'une des revendications 1 à 5, characterisée en ce que la proportion de la matière active et du solvent qui commande la libération de la matière active est entre 2:1 et 1:2.

7. Composition pharmaceutique selon la revendication 6, characterisée en ce que la proportion de la matière active et du solvent qui commande la libération de la matière active est entre 1:1 et 1:1,5.

8. Composition pharmaceutique selon l'une des revendications 1, à 7, characterisée en ce que le (les) solvent(s) pour la matrice qui s'évapore(nt) sur la peau est (sont) selectionné(s) de la groupe consistant de dichlorométhane, éthanol, éthylacetat, isopropanol ou mixtures de ces substances.

9. Composition pharmaceutique selon l'une des revendications 1 à 8, characterisée en ce que la proportion de la matrice polymerique et du solvent et du propellant est entre 1:10 et 1:15.

10. Composition pharmaceutique selon la revendication 9, characterisée en ce que la proportion de la matrice polymerique et du solvent et du propellant est entre 1:11 et 1:13.

11. Composition pharmaceutique selon l'une des revendications 1 à 10, characterisée en ce que dans la composition pharmaceutique le solvent ou la mixture des solvents, qui commande la libération de la matière active est contenue dans un contenu maximal de 35% rélative à la matrice polymère liquide, qui durcisse à un film flexible.

12. Composition pharmaceutique selon l'une des revendications 1 à 11, characterisée en ce que dans la composition pharmaceutique sont contenues de 2 à 10% des composants polymères qui durcissent à un film flexible, de 15 à 50% du solvent àvec un point d'ébullition bas, de 0,5 à 5% du solvent ou de la mixture des solvents qui commande la libération de la matière active et de 50 à 80% du propellant et la matière active.

13. Matrice pour une composition pharmaceutique qui a une activité transdermal thérapeutique pour l'application comme aérosol, characterisée en ce que la matrice contiens des composants polymères qui durcissent à un film flexible, comme des copolymères de vinylpyrrolidone et de vinylacetate et de l'ester butylique de l'acide polyméthacrylique, un solvent ou une mixture des solvents qui commande la libération de la matière active, qui est selectioné de la groupe consistant du macrogollaurate de sorbitane, paraffine, diglycérides ou triglycérides de l'acide gras à medium chaine, propylene carbonate ou mixtures de ces solvents et des solvents qui s'évaporent rapidement sur la peau et stimulent la préparation d'un film, selectionné de la groupe consistant de l'éthanol, dichlorométhane, éthylacetate, isopropanol ou mixtures de ces solvents.

14. Matrice pour une composition pharmaceutique qui a une activité transdermal thérapeutique pour l'application comme aérosol selon la revendication 13, characterisée en ce que la matrice contiens de 2 à 10% des composants polymères, qui durcissent à un film flexible, de 15 à 50% du solvent à point d'ébullition bas, de 0,5 à 5% du solvent ou de la mixture de solvents, qui commande la libération de la matière active, et de 50 à 80% du propellant.

15. Usage de la matrice selon l'une des revendications 13 ou 14 pour la préparation d'une composition pharmaceutique qui a une activité transdermal thérapeutique.